# EUROPÄISCHE PATENTANMELDUNG

(11) **EP 0 841 400 A1**
(43) Veröffentlichungstag der Anmeldung: **13.05.1998**
(21) Anmeldenummer: 96117908.2
(22) Anmeldetag: 08.11.1996
(51) Int. Cl.: C12N 15/66, C12N 15/70

(54) **Vektor zur bakteriellen Expression von rekombinanten Antikörper- und/oder Cytokin-Fusionstoxinen**

(71) Anmelder: Barth, Stefan, Dr. rer. nat., 50924 Köln (DE)
(72) Erfinder: Barth, Stefan, Dr. rer. nat., 50924 Köln (DE)
(74) Vertreter: Meyers, Hans-Wilhelm, Dr.Dipl.-Chem.

(57) **Zusammenfassung**

Vektor zur Expression rekombinanter Fusionstoxine aus einem Toxin und einem Liganden, der für bestimmte Tumorzellen spezifisch ist, mit einer für einen Bereich eines Antikörpers und/oder Cytokins codierenden Region und einer für ein Toxin oder den katalytisch wirksamen Bereich eines Toxins codierenden Region, weiteren Abschnitten zum Nachweis und zur Charakterisierung der rekombinanten Proteine sowie singulären Schnittstellen für Restriktionsenzyme,
dadurch gekennzeichnet, daß
- der Vektor eine multiple Klonierungsstelle (MCS-multiple cloning site) zur Insertion von variablen Antikörperregionen aufweist und mit einem Linker (Gly₄-Ser)₃ versehen ist,
- zu bekannten Bakteriophagensystemen (pCANTAB, pHEN) direkt kompatibel ist, mit der Maßgabe, daß die singulären Restriktionsschnittstellen der MCS in der codierenden Region des entsprechenden modifizierten Toxins nicht vorkommen.

## Beschreibung

Gegenstand der vorliegenden Erfindung ist ein Vektor zur bakteriellen Expression (E. coli) rekombinanter Fusionstoxine gemäß Oberbegriff des Patentanspruchs 1.

Auf der Suche nach alternativen Krebstherapien wurde für einen immuntherapeutischen Ansatz die bereits 1896 von Paul Ehrlich formulierte Vision von "Zauberkugeln (magic bullets)" aufgegriffen. Die heutigen Immuntoxine (ITs) werden durch chemische Kopplung eines pflanzlichen oder bakteriellen Toxins an einen tumorspezifischen Liganden hergestellt. Als Liganden werden monoklonale Antikörper oder Fab'-Fragmente verwendet. Die Entwicklung dieser Konstrukte begann vor etwa 15 Jahren mit dem Nachweis, daß ein IT gegen ein bestimmtes Oberflächenantigen einer spezifischen Tumorzelle diese selektiv zerstören kann. Seitdem wurde intensiv an der Entwicklung von ITs für den klinischen Einsatz gearbeitet.

Im Laufe der letzten zehn Jahre wurden zunehmend rekombinante Fusionstoxine konstruiert. Hierzu wird auf DNA-Ebene die codierende Region der unspezifischen Bindungsdomäne des eingesetzten zellulären Toxins durch Teile des Gens eines Antikörpers oder eines Cytokins ersetzt. Dabei können z. B. die variablen Regionen monoklonaler Antikörper nach PCR-Amplifikation über einen synthetischen Linker (Gly₄-Ser)₃ miteinander verbunden werden. Es entsteht ein einzelsträngiges variables Fragment (scFv), das ähnliche Affinität aufweisen kann wie der parentale Antikörper. Nach Ligation mit der codierenden Region der katalytisch-aktiven Domäne eines Giftstoffes wird ein einzelsträngiges chimäres Fusionstoxin exprimiert, das folgende Vorteile gegenüber chemisch gekoppelten ITs besitzt: 1. einfache großtechnische Herstellung, 2. Ermöglichung zielgerichteter Mutationen, 3. bessere Tumorpenetration aufgrund der geringen Molekülgröße.

Nachteilig an den hier zur Verfügung stehenden Vektorsystemen ist, daß diese nicht sauber induzierbar sind, d. h. auch ohne Inhibition der Repressorsysteme Fremdprotein produzieren, daß sie keine Möglichkeit des gerichteten Austauschs aller relevanten Sequenzabschnitte (Promotor, Aufreinigungs-Tag, Signalpeptid, Toxin) berücksichtigen und letztendlich keine direkte Klonierung von scFv-Genen aus bestehenden Bakteriophagensystemen ermöglichen.

Das der Erfindung zugrundeliegende technische Problem besteht darin, ein Vektorsystem zur Verfügung zu stellen, welches die oben beschriebenen Nachteile vermeidet.

Gelöst wird das der Erfindung zugrundeliegende technische Problem durch einen Vektor mit den Merkmalen des Patentanspruchs. Der erfindungsgemäße Vektor zur Expression rekombinanter Fusionstoxine ist aufgrund seiner Variationsmöglichkeiten vorteilhaft. Der Vektor eröffnet dadurch die Möglichkeit zur bakteriellen Expression beliebiger V-Genfragmente in die MCS, die gleichzeitig kompatibel zu bekannten Bakteriophagensystemen ist, so daß zusätzlich eine direkte Klonierung der scFv daraus ermöglicht wird, und das gekoppelt ist an entsprechend dieser MCS modifizierten Toxine, welche die entsprechenden Restriktionsschnittstellen der MCS nicht tragen.

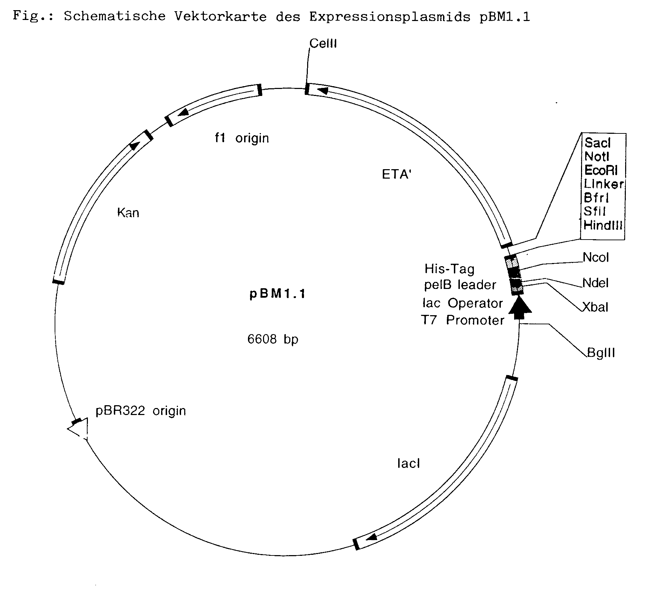

Vorzugsweise wird ein Konstrukt verwendet, wie es in der Figur dargestellt ist. Dieser Vektor ermöglicht die Produktion von rekombinanten Fusionstoxinen in Escherichia coli. Das Ausgangsplasmid für die Konstruktion des Vektors gemäß Figur 1 war das pET27b der Firma Novagen (Madison, USA). Der beschriebene Vektor pBM1.1 ist ausgestattet mit mehreren singulären Restriktionsschnittstellen, die den Einbau variabler codierender Regionen ermöglichen.
1. Promoter (BglII, XbaI), bspw. T7, lac, tac,
2. Signalpeptid (XbaI, NdeI), bspw. pelB, ompA, phoA,
3. Selektionsregion (NcoI, HindIII), bspw. Flag, His, Cal, Myc, E,
4. variable Ig-Regionen, (HindIII, BfrI und EcoRI, SacI),
5. scFv-Gene aus gängigen Bakteriophagensystemen (SfiI, NotI),
6. Toxine (SacI, CelII), bspw. ETA', Ricin, DT, EDN, Angiogenin.

Die in diesem Vektor verwendeten Toxine dürfen die singulären Schnittstellen der MCS nicht aufweisen. Das inserierte ETA' ist eine Deletionsmutante des Pseudomonas aeruginosa Exotoxin A, dem die ursprüngliche (unspezifische) Bindungsdomäne entfernt worden ist und dessen interne SfiI-Schnittstellen ebenfalls zerstört wurden.

Das hier beschriebene Vektorsystem besitzt eine MCS, die sich 1. dadurch auszeichnet, daß sie mit Restriktionsschnittstellen versehen wird, die einerseits mit einer sehr geringen Wahrscheinlichkeit in den amplifizierten V-Genen und andererseits nicht in der codierenden Region des Toxins vorhanden sind.

**Tabelle**

| "sticky end" Restriktionsenzyme die < 10% der untersuchten V-Gene schneiden* | |
|---|---|
| Vh | Vl |
| ApaI, ApaLI, BamHI, BclI, BfrI, BglII, BsiWI, BstBI, BstXI, Bsu36I, ClaI, EclXI, EcoRI, HindIII, KpnI, MluI, NarI, NcoI, NdeI, NheI, NotI, SacI, SacII, SalI, Van91I, XbaI, XhoI | ApaI, ApaLI, BfrI, BclI, BglII, BsiWI, BstBI, BspHI, Bsu36I, ClaI, EclXI, EcoRI, EspI, HindIII, MluI, NarI, NdeI, NheI, SacI, SacII, SalI, |

| | |
|---|---|
| + nach Chadhary et al., 1990 | |

Da die verwendete Deletionsmutante des Pseudomonas Exotoxin A (ETA') nicht von den Enzymen HindIII, BfrI, EcoRI und SacI geschnitten wird, wurden die Konsensussequenzen dieser Restriktionsenzyme für den Aufbau der MCS verwendet. Um die V-Genfragmente zu einem funktionellen scFv zusammensetzen zu können, wurde noch ein (Gly₄-Ser)₃-Linker zwischen BfrI und EcoRI eingefügt.

Als zweites Charakteristikum der MCS sind die Konsensussequenzen der Enzyme SfiI und NotI eingefügt, die eine direkte Klonierung von scFv aus kommerziell verfügbaren Bakteriophagensystemen ermöglicht.

Das verfügbare ETA' ist überraschenderweise mit zwei SfiI-Schhittstellen bestückt, die eine direkte Klonierung der scFv aus bekannten Phagmiden unmöglich machen. Deswegen wurden diese Schnittstellen und zusätzlich eine XhoI-Schnittstelle durch Mutagenese in entsprechenden Wobble-Basen, ohne Auswirkung auf die primäre Aminosäuresequenz verändert.

Sollten weiter zu verwendende Toxine ebenfalls interne Restriktionsschnittstellen aufweisen, wie sie in der MCS verwendet werden, kann letztere bezüglich der Tabelle variiert werden; sollte sich eine Konsensussequenz für SfiI oder NotI im Toxin befinden, so muß diese ebenfalls durch Punktmutationen entfernt werden.

Die Konstruktion des beschriebenen Vektors erfolgt nach an sich bekannten molekularbiologischen Verfahrensweisen.

## Patentansprüche

1. Vektor zur Expression rekombinanter Fusionstoxine aus einem Toxin und einem Liganden, der für bestimmte Tumorzellen spezifisch ist, mit einer für einen Bereich eines Antikörpers und/oder Cytokins codierenden Region und einer für ein Toxin oder den katalytisch wirksamen Bereich eines Toxins codierenden Region, weiteren Abschnitten zum Nachweis und zur Charakterisierung der rekombinanten Proteine sowie singulären Schnittstellen für Restriktionsenzyme,
dadurch gekennzeichnet, daß
- der Vektor eine multiple Klonierungsstelle (MCS-multiple cloning site) zur Insertion von variablen Antikörperregionen aufweist und mit einem Linker (Gly₄-Ser)₃ versehen ist,
- zu bekannten Bakteriophagensystemen (pCANTAB, pHEN) direkt kompatibel ist, mit der Maßgabe, daß die singulären Restriktionsschnittstellen der MCS in der codierenden Region des entsprechenden modifizierten Toxins nicht vorkommen.
